# EUROPÄISCHE PATENTSCHRIFT

(11) **EP 3 388 003 B1**
(45) Veröffentlichungstag und Bekanntmachung des Hinweises auf die Patenterteilung: **22.07.2020**
(21) Anmeldenummer: 17020149.5
(22) Anmeldetag: 12.04.2017
(51) Int. Cl.: A61B 17/225, A61H 23/00, A61H 23/04, A61B 17/00

(54) **DRUCKWELLENGERÄT**
PRESSURE WAVE DEVICE
APPAREIL D'ONDE DE PRESSION

(43) Veröffentlichungstag der Anmeldung: 17.10.2018
(73) Patentinhaber: STORZ MEDICAL AG, 8274 Tägerwilen (CH)
(72) Erfinder: Novak, Pavel, 8234 Stetten (CH); Schulz, Manfred, 2374 Tägerwilen (CH); Swart, Stephan, 47447 Moers (DE); DiMaio, Carlo, 47199 Duisburg (DE)
(74) Vertreter: König Szynka Tilmann von Renesse Patentanwälte Partnerschaft mbB

(56) Entgegenhaltungen:
- WO-A1-2014/036170
- DE-A1- 10 215 416
- US-A- 5 941 838
- US-A1- 2014 350 438

## Beschreibung

Die vorliegende Erfindung bezieht sich auf ein Gerät zur Behandlung des menschlichen oder tierischen Körpers durch Ausüben von Stößen auf die Körperoberfläche. Im Folgenden wird zur Vereinfachung vom Körper eines Patienten gesprochen, der bevorzugt menschlich ist.

Im Stand der Technik sind verschiedene Geräte des beschriebenen Grundtyps bekannt. Die DE 197 25 477 C beschreibt bspw. ein solches Gerät, bei dem durch die Kollision eines pneumatisch beschleunigten Schlagteils oder Projektils mit einem zunächst ruhenden Prallkörper oder Applikator eine Druckwelle ausgelöst wird, die dadurch in den Körper des Patienten eingekoppelt werden kann, dass eine Applikatorfrontfläche zum Zeitpunkt der Kollision auf den Patientenkörper aufgelegt wird. Dieser Gerätetyp leitet sich in seiner Entstehungsgeschichte von Lithotripsiegeräten ab, bei denen solche Druckwellen z. B. über eine lange stabförmige Sonde an der Frontfläche des Applikators auf einen Nierenstein oder Ähnliches übertragen werden können, um diesen zu desintegrieren.

Der Schwerpunkt bei der Darstellung liegt in jedem Fall auf der durch die Kollision erzeugten Druckwelle, die mehr oder weniger einer eigentlichen Stoßwelle ähneln soll, wie sie klassische, in der Regel fokussierende Lithotripsiegeräte z. B. mit piezoelektrischen oder elektromagnetischen Aktuatoren erzeugen. Solche Druckwellen können Anstiegsflanken mit einer Breite im Bereich weniger µs und einer Amplitude im niedrigen zweistelligen MPa-Bereich haben (z. B. 2 µs und 15 MPa gemessen 1 cm vor der Frontfläche). In dem zitierten Dokument wird hingegen betont, dass die physikalisch an sich unvermeidliche makroskopische Schwerpunktbewegung des Applikators möglichst klein gehalten werden soll, weil sie als störend angesehen wird.

Als zweites Beispiel wird auf die DE 20 2004 011 323 U und, mit sehr ähnlichem Inhalt, die US 2011/0054367 A1 verwiesen. Dort wird ein hinsichtlich des technischen Aufbaus ähnliches Gerät beschrieben, bei dem allerdings u. a. die elastische Aufhängung des Applikators im Gehäuse auf größere Schwerpunktsbewegungen des Applikators ("Hübe") ausgelegt ist. Dort wird betont, dass die therapeutische Wirkung durchaus auch, oder vorwiegend in den eigentlichen makroskopischen Stößen (also infolge des Hubes) gesehen werden kann, was natürlich auch von der Indikation abhängt.

Die vorliegende Erfindung richtet sich allgemein auf Geräte dieses Bautyps, und zwar sowohl hinsichtlich der Anwendung von Druckwellen als auch hinsichtlich der Anwendung "makroskopischer Stöße" des Applikators auf die Körperoberfläche.

Zum Stand der Technik wird ergänzend verwiesen auf die WO 2014/036170 A1. Dort geht es um Behandlungsköpfe für Ultraschallgeräte zur Hirnbehandlung, in denen Kopplungskörper aus Silikonmaterial Verwendung finden.

Ferner wird verwiesen auf die US 2014/350438 A1, die ein Gerät nach dem Oberbegriff des Anspruchs 1 offenbart, der es dabei aber um die Erzeugung und Einkopplung besonders starker Stoßwellen (infolge ungewöhnlich großer pneumatischer Versorgungsdrücke eines ballistischen Mechanismus) geht. Mit konkaven Frontflächen sollen die Stoßwellen fokussiert werden, um Lithotripsie betreiben zu können. Zur Impedanz- und Formanpassung sind vor den konkaven Frontflächen flüssigkeitsgefüllte Kissen vorgesehen.

Die US 5,941,838 A beschreibt ebenfalls ein fokussierendes Stoßwellengerät, allerdings mit einem elektromagnetischen Mechanismus mit einer konkaven Spule, die in ein Silikonmaterial eingebettet ist, das auf seiner Innenseite eine Metallmembran trägt, an die sich ein wasser- oder gelgefülltes Kissen zur Ankopplung an den Körper anschließt. Das die Spule tragende Silikonmaterial kann eine Shore-A-Härte zwischen 30 und 60 haben.

Schließlich wird verwiesen auf die DE 102 15 416 A1, die ein ballistisches Druckwellengerät mit einer speziellen Applikatorform zeigt. Diese Form bildet einerseits einen Exponentialtrichter und andererseits eine fokussierende konkave Frontfläche, die zur Vermeidung von Lufteinschlüssen durch einen Einsatz (mit seinerseits planer Frontfläche) gefüllt werden kann.

Der Erfindung liegt dabei die Aufgabe zugrunde, ein eingangs beschriebenes Gerät hinsichtlich weitergehender Anwendungsmöglichkeiten auf der Patientenkörperoberfläche weiterzubilden.

Erfindungsgemäß löst diese Aufgabe ein Gerät zur Behandlung des menschlichen oder tierischen Körpers mit einem Applikator zum Auflegen auf den Körper von außen, einem Gehäuse, in dem der Applikator gehalten ist, und einem Mechanismus zum Erzeugen von Stößen des Applikators relativ zu dem Gehäuse in einer Stoßrichtung, so dass die Stöße bei dem Auflegen in den Körper eingekoppelt werden können, dadurch gekennzeichnet, dass der Applikator an einer in der Stoßrichtung nach vorn weisenden und zum Auflegen auf den Körper vorgesehenen Frontfläche hohl und weich ausgestaltet ist und die weiche Ausgestaltung sich daraus ergibt, dass der Applikator an der hohlen und weichen Frontfläche aus einem Elastomermaterial mit einer Shore-Härte von höchstens 60 Sh besteht, wobei das Elastomermaterial an der Hohlform eine Stärke in der Stoßrichtung von mindestens 3 mm aufweist, wobei 5 mm, 7 mm, 10 mm als untere Grenze bevorzugt sind.

Bevorzugte Ausgestaltungen der erfindungsgemäßen Vorrichtung sind in den abhängigen Ansprüchen angegeben. Die darin enthaltenen Merkmale und auch die Offenbarung der folgenden Beschreibung sind grundsätzlich im Hinblick auf beide Erfindungskategorien zu verstehen, ohne dass hierzwischen immer im Einzelnen explizit unterschieden wird.

Die Grundidee der Erfindung liegt darin, den die Stöße auf den zu behandelnden Körper ausübenden Applikator an seiner auf den Körper aufzulegenden Frontfläche hohl und besonders weich auszugestalten. Damit lässt sich der Applikator nach den Erkenntnissen der Erfinder besonders effektiv und patientenschonend einsetzen, und zwar insbesondere auf durch die Körperoberfläche leicht zu ertastenden bzw. nahe darunterliegenden Knorpel- oder Knochenteilen, etwa Wirbeln.

Die Hohlform verhindert bei möglicherweise größerem Anpressdruck, ungünstiger Haltung der behandelnden Person, unbeabsichtigten Bewegungen des Patienten und/oder rutschigem Kopplungsgel oder Schweiß zwischen der Frontfläche und dem Körper ein Abrutschen zur Seite. Sie sorgt ferner für eine gute geometrische Anpassung an vorstehende Knorpel- oder Knochenformen, wie etwa bei Wirbeln.

Die weiche Ausgestaltung schont dabei den Knorpel oder den Knochen und auch das darüberliegende Gewebe und die Haut und koppelt die Stöße (und optional auch die eingangs beschriebenen Druckwellen) besonders schonend in den Körper ein. Konkret hat sich dabei eine Shore-Härte von höchstens 60 Sh bewährt. Die Shore-Härte ist die übliche Größe zur Beschreibung der Härte von Elastomeren und wird in der Einheit Shore (Sh) gemessen. Üblicherweise geht man dabei von Raumtemperatur aus (23° C), und im vorliegenden Fall wird auf die Messung Shore-A abgestellt.

Besonders bevorzugterweise liegt die Shore-Härte bei höchstens 55 Sh, 50 Sh, 45 Sh oder sogar nur höchstens 40 Sh.

Das Elastomermaterial des Applikators mit dieser Shore-Härte liegt an der beschriebenen Hohlform (und optional auch darüber hinaus) vor, hat aber erfindungsgemäß an der Hohlform eine Stärke in der Stoßrichtung von vorzugsweise mindestens 3 mm, um die mechanischen Eigenschaften gut ausspielen zu können, wobei als untere Grenze 4 mm, 6 mm, 8 mm, 10 mm besonders bevorzugt sind.

Die beschriebene Hohlform soll bedeuten, dass mittlere Bereiche der Frontfläche gegenüber eher am Rand der Frontfläche liegenden Bereichen zurückspringen (bei nach oben weisender Frontfläche würde also Flüssigkeit in der Hohlform verbleiben). Auf geometrische Einzelheiten wird weiter unten eingegangen.

Ähnlich wie bei der DE 197 25 477 C und der DE 20 2004 011 323 U ist auch bei dieser Erfindung ein sogenannter ballistischer Mechanismus bevorzugt, bei dem die Stöße durch die Kollision eines Projektils mit dem Applikator erzeugt werden, üblicherweise auf einer relativ zu der Frontfläche entgegengesetzten Kollisionsfläche des Applikators. Das Projektil kann in unterschiedlicher Weise beschleunigt werden, wobei eine pneumatische Beschleunigungseinrichtung besonders bevorzugt ist.

Der Applikator kann im Einzelfall ganz aus dem relativ weichen Elastomermaterial bestehen, wenn der Einkopplung von Druckwellen im Sinn von durch eine harte Festkörperkollision bedingten Schallwellen nicht gewünscht ist. Vorzugsweise allerdings werden für den Applikator mindestens zwei Materialien eingesetzt, von denen das härtere die Kollisionsfläche bildet, auf die das Projektil aufschlägt. Diese zweite Materialwahl sollte vorzugsweise ein Material betreffen, das ebenfalls bei der Kollisionsfläche eine Stärke in der Stoßrichtung von mindestens 5 mm hat. Vorzugsweise ist das Material nicht-elastomer.

In Betracht kommen hier z. B. Metalle, insbesondere rostfreier Stahl. Daneben sind als metallische Materialien Aluminium und Titan zu nennen, wobei beide eine relativ geringe Massendichte haben und damit relativ leichte Applikatoren ermöglichen. Das kann den Vorteil haben, bei gegebener Geometrie eine stärkere Beschleunigung und damit auch größere Hubauslenkung des Applikators zu ermöglichen, was im vorliegenden Fall gewünscht sein kann. Titan zeichnet sich zudem durch eine besonders hohe mechanische Belastbarkeit aus, eignet sich also besonders für Anwendungen mit vergleichsweise hohen Projektilgeschwindigkeiten und/oder Projektilmassen. In Betracht kommen, insbesondere für das drehbare Applikatorteil, ferner Keramiken, vgl. EP 08 003 840.9 / EP 2 095 843, und Kunststoffe. Kunststoffe kommen insbesondere auch dann in Betracht, wenn auf die Einkopplung einer Druckwelle geringerer Wert gelegt wird, weil, jedenfalls bei einer größeren Stärke des Kunststoffs, im Vergleich zu den vorgenannten Materialien etwas größere Wellenleitungsverluste zu erwarten sind. Gleiche Argumente gelten für Holz.

Bei z. B. elektromagnetischer Beschleunigung kommen natürlich primär Metalle in Betracht und die pneumatische Beschleunigung ist diesbezüglich flexibler.

Eine bevorzugte Wahl für das weiche Material des Applikators an der Frontfläche ist Silikonkautschuk, der auch eine gute Verträglichkeit für den Patienten aufweist.

Der Applikator hat vorzugsweise eine rotationssymmetrische Form bzgl. einer Symmetrieachse, die der Stoßrichtung entspricht bzw. zu ihr parallel ist, wobei diese Achse außerdem die Längsachse des Applikators und die Beschleunigungsrichtung des Projektils sein kann. Die Symmetrieanforderung gilt dann zumindest für den sichtbaren Teil des Applikators, also den außerhalb eines Gehäuses des Geräts liegenden Teil und damit den für die behandelnde Person und den Patienten primär relevanten Teil. Das Gerät kann dann z. B. während der Behandlung ohne Weiteres gedreht werden.

Der Applikator kann ferner in seinem vorderen, also distalen und dem Patienten zugewandten Bereich verjüngt geformt sein, insbesondere konisch, und sich dementsprechend auf die Frontfläche zu im Durchmesser oder einer mittleren Querabmessung verringern. Das kann z. B. mit einem halben Öffnungswinkel von 10° bis 15° der Fall sein. Vorzugsweise ist die gesamte Mantelfläche des weichen Applikatorteils davon betroffen, also mit Ausnahme der Frontfläche.

Das weiche Material kann, wie oben schon dargelegt, an einem weiteren Applikatorteil aus härterem Material gehalten sein, und zwar insbesondere in einem Hinterschnitt (bzgl. Trennkräften in Stoßrichtung). Insbesondere kann das Material eines zweiten Applikatorteils mit der Kollisionsfläche eine hinterschnittene Hohlform aufweisen, in die das weichere Material, z. B. Silikonkautschuk, eingedrückt oder eingefüllt ist. Ferner kann dazwischen eine die Haftung vergrößernde Beschichtung vorgesehen sein.

Die Hohlform der Frontfläche ist vorzugsweise relativ deutlich ausgeprägt, und zwar vorzugsweise mit einer Tiefe von 10 % bis 30 % des gemittelten Durchmessers der Hohlform senkrecht zur Stoßrichtung, wobei als Obergrenze 25 % und als Untergrenze 15 % besonders bevorzugt sind. Dies gewährleistet eine gute Verankerung bei der Anwendung und ein gutes Umschließen z. B. von hervorstehenden Formen von Wirbelknochen.

Ferner ist die Hohlform vorzugsweise rundlich konkav, also in einen die Stoßrichtung enthaltenden Schnitt rundlich. Besonders bevorzugterweise ist sie dabei sphärisch, wobei bevorzugte Krümmungsradien zwischen 5 mm und 20 mm liegen. Als Untergrenze sind 6 mm und 7 mm und als Obergrenze 17 mm, 14 mm, 12 mm und 10 mm besonders bevorzugt.

Da der Applikator mit einer gewissen Kraft aufgesetzt werden soll, kann es von Vorteil sein, nicht allzu weiche Materialien einzusetzen. Werte von mindestens 10 Sh, besser mindestens 15 Sh oder 20 Sh, sind bevorzugt.

Nach einer weiteren Ausgestaltung ist der Applikator in einer lösbaren Kupplung gehalten, sodass zumindest ein distales Applikatorelement (mit dem drehbaren Applikatorteil) aus dem Gerät nach vorn herausgezogen werden kann. (Zur Klarstellung: Die Begriffe "proximal" und "distal" werden hier aus der Perspektive eines Benutzers des Geräts gebraucht; in anderen Worten bedeutet "distal" eine dem Patienten zugewandte Position und "proximal" eine dazu entgegengesetzte.)

Die Erfinder haben festgestellt, dass die Optimierung der Applikatoreigenschaften im Hinblick auf bestimmte Behandlungen, bspw. der Wechsel zwischen einem erfindungsgemäßen Applikator und einem konventionellen oder zwischen verschiedenen erfindungsgemäßen Applikatoren, vorteilhafterweise mit im Übrigen unverändertem Gerät von Fall zu Fall vorgenommen werden kann.

Ein Applikator kann zu diesem Zweck mehrteilig aufgebaut sein, wobei die Entnehmbarkeit primär ein distales Element betrifft. Dieses distale Element des Applikators kann dann gegen andere entsprechende distale Applikatorelemente getauscht werden, zum Beispiel weil das Applikatorelement nur eine begrenzte Lebensdauer hat (etwa wegen des weichen Materials), weil es gereinigt oder sterilisiert werden soll oder weil ein anderer Typ dieses distalen Applikatorelements (oder des gesamten Applikators) verwendet werden soll. Insbesondere ist es möglich, in ihrem distalen Teil unterschiedlich geformte Applikatorelemente, möglicherweise auch aus verschiedenen Materialien, einzusetzen.

Der Austausch erfolgt dabei sehr einfach und schnell, vorzugsweise völlig werkzeuglos und ebenfalls vorzugsweise ohne Abbauen eines weiteren Geräteteils, etwa einer Aufschraubkappe bei konventionellen Geräten dieses Typs.

Eine bevorzugte Ausführungsform sieht eine Verdrehsicherung des Applikators in dem Gerät vor (nicht zwingend in der Kupplung). Dementsprechend kann zum Beispiel die Kupplung selbst eine Verdrehsicherheit herstellen oder durch Einschieben eines entsprechend gestalteten Bereichs des Applikators in eine entsprechende Aufnahme eine Verdrehsicherheit hergestellt werden, besonders bevorzugterweise durch einen Mehrkantstift auf der Applikatorseite und eine dazu passende Aufnahme auf der Geräteseite, etwa durch einen hexagonalen Formschluss.

Die Kupplung kann vorzugsweise ebenfalls mit einem Formschluss arbeiten, der nicht zwingend mit dem Formschluss für die Verdrehsicherung übereinstimmen muss. Insbesondere kann eine Klemmhülse vorgesehen sein, die (vorzugsweise in Stoßrichtung) verschiebbar ist und dabei zumindest ein Formschlusselement in eine entsprechende Aufnahme an dem Applikator hereindrückt bzw. beim Lösen freigibt. Insbesondere kann ein Gehäuseteil des Geräts gegen eine Federkraft verschiebbar sein und dabei die Klemmhülse aufweisen oder mitverschieben. Als Formschlusselement kommt eine Kugel in Betracht, wobei vorzugsweise mindestens zwei Kugeln vorgesehen sind. Die Kugeln oder andere Formschlusselemente können durch eine schräge Fläche an einer Innenseite der Klemmhülse beaufschlagt werden.

Im Folgenden wird die Erfindung auch anhand eines Ausführungsbeispiels näher erläutert, dessen einzelne Merkmale im Rahmen des geltenden Anspruchs 1 auch unabhängig voneinander und in anderen Kombinationen erfindungswesentlich sein können.
- Figur 1: zeigt ein Druckwellengerät als erstes Ausführungsbeispiel der Erfindung;
- Figur 2: zeigt ein zweites Ausführungsbeispiel, wobei das Druckwellengerät nur ausschnittsweise und hinsichtlich einer im ersten Ausführungsbeispiel nicht enthaltenen Applikatorkupplung dargestellt ist;
- Figuren 3 und 4: zeigen auswechselbare Applikatorelemente für das zweite Ausführungsbeispiel in Figur 2.

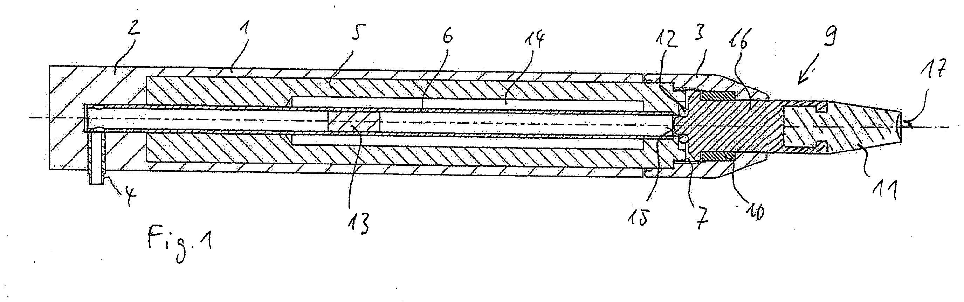

Figur 1 zeigt ein erstes Ausführungsbeispiel der Erfindung. Es handelt sich um ein Gerät zur Einkopplung von Stößen und unfokussierten (so genannten radialen) mechanischen Druckwellen in den menschlichen oder tierischen Körper.

Ein Rohrstück 1 bildet gemeinsam mit einer in der Anwendung körperabgewandten und mit dem Rohrstück 1 integrierten Zuluftkappe 2 und einer in der Anwendung körperzugewandten Applikatorkappe 3 ein Gehäuse. Die Zuluftkappe 2 enthält einen Druckluftanschluss 4 für eine pneumatische Versorgung. In an sich bekannter Weise ist an diesen Druckluftanschluss 4 über eine pneumatische Versorgungsleitung ein von einer Ansteuereinheit gesteuertes Ventil, insbesondere Magnetventil, angeschlossen, das in einem gleichbleibenden iterativen Takt zwischen etwa 1 Hz und 50 Hz Druckluftpulse über den Druckluftanschluss einkoppelt. Das Ventil ist nicht gezeigt und kann auch in dem dargestellten Gerät selbst eingebaut sein.

Das Gerät ist im Übrigen als mit der Hand einer Bedienungsperson zu haltendes Gerät ausgebildet, das über die erwähnte Pneumatikleitung an eine nicht gezeigte Basisstation mit der Ansteuereinheit und dem Kompressor angeschlossen ist und auf den Patienten manuell aufgesetzt werden kann. Es eignet sich besonders für die Behandlung von Körperpartien hinter körpereigenen Hindernissen wie Rippen oder Schulterblättern.

In dem Gehäuse ist über einen Einsatz 5 ein Führungsrohr 6 gehalten, dessen bei der Anwendung körperfernes Ende in der Zuluftkappe 2 endet und dort mit dem Druckluftanschluss 4 kommuniziert. Das in der Anwendung körperseitige Ende des Führungsrohres 6 endet in einem Teil des Einsatzes 5, der in die Applikatorkappe 3 hineinragt, und zwar kurz vor dem dortigen Ende des Einsatzes 5 und einem Innenraum 7 in der Applikatorkappe 3.

In dem Innenraum 7, der in eine in der Anwendung körperseitige Applikatoröffnung übergeht, ist ein erster und in Figur 1 linker Teil eines Applikators 9 aufgenommen, und zwar aus einem harten Material, hier Edelstahl. Dieser stützt sich über ein elastisches Schlauchelement 10 aus einem Elastomer an einer radialen Schulter ab. Ein zur körperfernen Seite gerichtetes und die Kollisionsfläche 15 beinhaltendes Ende des Applikators 9 stützt sich über einen O-Ring 12 an dem Einsatz 5 ab, und zwar an einer das bereits erwähnte Ende des Einsatzes 5 umgebenden Stirnfläche. Dabei liegt der O-Ring 12 zwischen dieser Stirnfläche und einer Schulter des Applikators 9. Die Applikatoröffnung 8 dient dabei zu einer in der Längsrichtung verschiebbaren Führung des Applikators 9 und fixiert diesen quer zur Längsrichtung. Die Axialverschiebbarkeit ist nur durch die Nachgiebigkeit des Elastomerelements 10 begrenzt und kann bei einem in Luft betriebenen Gerät relativ zum Restgerät auch deutlich über 1 mm liegen.

Der Applikator 9 weist als zweiten Teil das in Figur 1 rechte Element 11 auf, das den eigentlichen auf die Haut aufzusetzenden Applikatorteil bildet und aus Silikonkautschuk mit einer Härte von etwa 30 Sh besteht. Der Applikatorteil 11 ist dabei in eine innen hinterschnittene Hohlform des Applikatorteils 16 eingegossen.

Der Applikator 9 ist durch Abschrauben der Applikatorkappe 3 austauschbar.

In dem angrenzenden Bereich des Führungsrohres 6 ist ein in Figur 1 mit dem Applikator 9 in Kontakt stehendes Projektil 13 eingesetzt. Dieses passt (in Bezug auf das Führungsrohr und die im Wesentlichen zylindrische Geometrie des Projektils 13) radial mit geringem Spiel hinein. Das Projektil 13 kann durch Druckunterschiede der Luftsäule in dem Führungsrohr 6 vor und hinter ihm (d. h. in Figur 1 rechts und links des Projektils 13) in dem Führungsrohr hin- und herbewegt werden und insbesondere auf den Applikator 9 zu beschleunigt werden. Hierzu wird es aus einer Ausgangsposition (nicht gezeigt) in Figur 1 links durch einen Druckluftstoß durch den Druckluftanschluss 4 beschleunigt und trifft mit seiner dem Applikator 9 zugewandten Frontfläche auf den Applikator 9 auf, und zwar auf eine körperabgewandte Prallfläche 15 davon.

Die Rückbewegung des Projektils 13 erfolgt zusätzlich zu einem Zurückprallen nach der Kollision durch ein Rückströmen der Luft aus einer das Führungsrohr 6 innerhalb des Einsatzes 5 umgebenden Staukammer 14. In diese wird die Luft bei der Beschleunigung des Projektils 13 in Richtung zu dem Prallkörper 9 verdrängt und damit dort komprimiert. Wenn das Magnetventil den Druck wegschaltet und gleichzeitig den Raum hinter dem Projektil entlüftet, wird das Projektil 13 damit in die Ausgangsstellung zurückbewegt. Dies kann natürlich auch durch eine zusätzliche oder alternative Druckbeaufschlagung der Staukammer 14 oder eines anderen Luftvolumens körperseitig von dem Projektil 13 erfolgen. Das in der Anwendung körperferne Ende des Führungsrohres 6 endet in einem Magnethalter für das Projektil 13.

Man erkennt, dass der Applikator 9 hinsichtlich seiner Längserstreckung in Stoßrichtung ungefähr hälftig (unter Nichtberücksichtigung der Hohlform) aus dem "harten" linken Applikatorteil 16 und hälftig aus dem "weichen" rechten Applikatorteil 11 besteht, wobei beide eine axiale Länge von ungefähr 35 mm und einen Maximaldurchmesser von ungefähr 20 mm haben. Etwa vom distalen Ende der Hohlform ausgehend verjüngt sich der weiche Applikatorteil 11 konisch bis auf einen Durchmesser von etwa 15 mm über eine Länge von hier etwa 15 mm (nicht maßstäblich gezeichnet). Die sich daran anschließende Frontfläche 17 ist sphärisch konkav mit einem Krümmungsradius von etwa 8 mm und damit einer mittigen Tiefe (gegenüber einer fiktiven planen Frontfläche) von etwa 3 mm. Der Rand ist verrundet mit einem Radius von 0,7 mm.

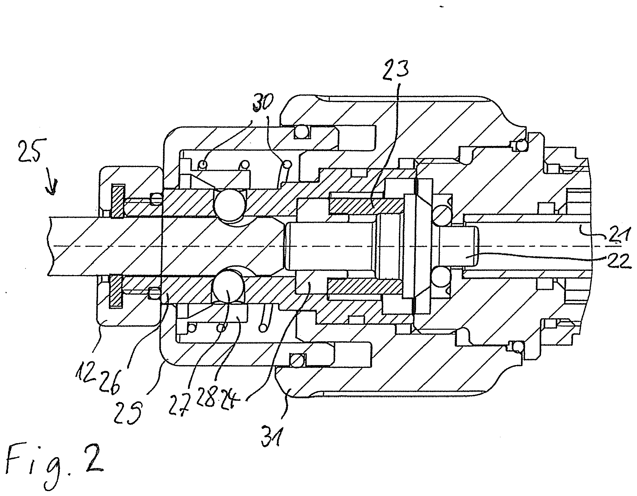

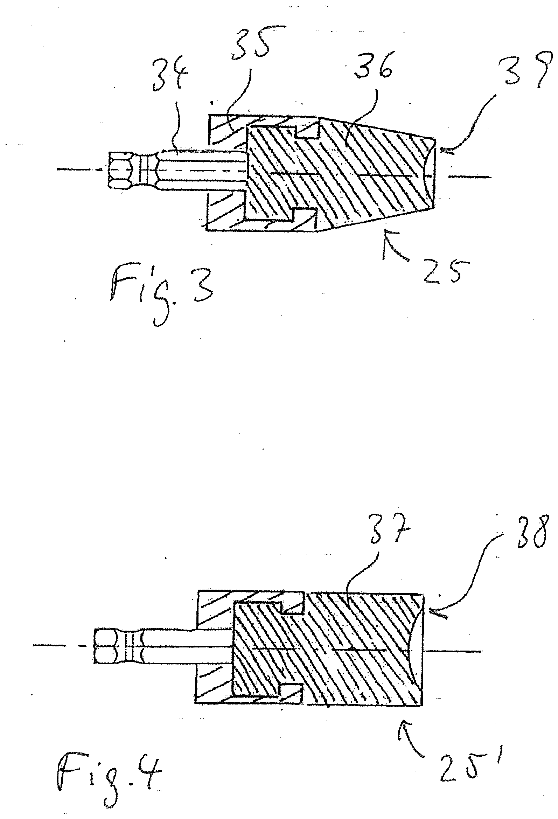

Figur 2 zeigt ein zweites Ausführungsbeispiel, bei dem (gegenüber Figur 1 rechtslinks-vertauscht) das applikatorseitige Ende eines ansonsten Figur 1 entsprechenden Geräts dargestellt ist, das jedoch mit einer speziellen Kupplung zur Aufnahme besonders leicht wechselbarer Applikatorelemente vorgesehen ist. Die folgenden Figuren 3 und 4 zeigen zwei hier einsetzbare Applikatorelemente. Doch zunächst zu Figur 2: Ein proximales, also in Figur 2 rechtes Applikatorelement 22 hat eine im Vergleich zum Stand der Technik und zu Figur 1 weitgehend konventionelle Form und stützt sich dementsprechend mit einem radial vergrößerten Flansch und dessen distaler Schulterseite an einem Elastomerschlauchstück 23 ab, das bei den Kollisionen verformbar ist und eine Bewegung des proximalen Applikatorteils 22 im Bereich über einem Millimeter zulassen kann. Es ist ferner für die Rückstellung verantwortlich und entkoppelt die Schläge vom Gerätegehäuse. An der distalen Seite läuft dieses proximale Applikatorelement 22 in einem zylindrischen Stift aus, der eine Führungsmanschette 24 durchsetzt und zur distalen Seite etwas darüber hinausragt. Bei konventionellen Geräten wäre die distale Stirnfläche dieses Stifts die Kontaktfläche des Applikators zum Patienten und die Führungsmanschette 24 in einer (oder als) abschraubbaren Kappe realisiert. Man müsste also die Kappe abnehmen und damit eine Teilzerlegung vornehmen, bevor man den Applikator (in diesem Fall das proximale Applikatorelement 22) entnehmen kann. Analoges gilt für die Variante aus Figur 1 mit dem Applikator 9.

Figur 2 zeigt ferner, dass sich die linke Stirnfläche des proximalen Applikatorelements 22 in Kontakt mit der rechten Stirnfläche eines distalen Applikatorelements 25 befindet. Auf dieses distale Applikatorelement 25 wird anhand der Figuren 3 und 4 näher eingegangen. Es ist in einem Aufnahmestück 26 geführt, und zwar formschlüssig, z. B. hexagonal, und damit gegenüber diesem Aufnahmestück 26 gegen Verdrehung gesichert. Das Aufnahmestück 26 seinerseits muss entsprechend fest verdrehgesichert im übrigen Gehäuse gehalten sein.

Figur 2 zeigt ferner, dass in dem Aufnahmestück 26 (in Figur 2 oben und unten) Öffnungen für Formschlusskugeln 27 vorgesehen sind, die nach innen in entsprechende außenseitige Nuten an dem distalen Applikatorteil 25 eingreifen. Diese Nuten sind in der Figur nach rechts länger als nötig, sodass sich der gesamte Applikator 25 und 26 bei einem Stoß nach links bewegen kann. Andererseits verhindert der Formschluss zwischen den Kugeln 27 und den dazugehörenden Nuten ein Herausfallen oder -schießen des distalen Applikatorelements aus dem Gehäuse. Das distale Applikatorelement kann vielmehr zum Beispiel einfach durch Aufdrücken auf den Patienten nach einem Stoß wieder in seine gezeichnete Ursprungslage zurückgeschoben werden.

Radial außerhalb der Kugeln 27 erkennt man eine Klemmhülse 28, die ersichtlich an ihrem linken Ende eine innere Schrägfläche aufweist. Wenn diese Klemmhülse 28 aus der gezeichneten Position heraus nach rechts verschoben wird, können die Kugeln 27 radial nach außen weichen und kann das distale Applikatorteil 25 insgesamt einfach nach links aus dem Gehäuse herausgezogen werden. Für dieses Verschieben der Klemmhülse 28 sorgt ein händisches (nach rechts) Hineindrücken eines von außen zugänglichen Gehäuseteils 29, wobei eine Schraubenfeder 30 dagegen drückt bzw. für die Rückführbewegung sorgt und dieses verschiebbare Gehäuseteil 29 in einem weiteren Teil 31 des Gehäuses verschiebbar und durch einen O-Ring gehemmt geführt ist.

In Figur 2 links von dem verschiebbaren und zur Betätigung der Kupplung dienenden Gehäuseteil 29 findet sich noch eine Sicherungsmutter 32, die auf dem Aufnahmeteil 26 aufgeschraubt und gegenüber diesem über einen O-Ring und gegenüber dem Teil 25 über einen Elastomerflachring abgedichtet ist.

Insgesamt erkennt man, dass ein Projektilstoß den gesamten Applikator nach links versetzt und dabei eine entsprechende Stoßbewegung des nicht gezeichneten distalen Endes des Applikators (ganz links) gegen den Patientenkörper zur Folge hat, wobei die Einkopplung einer eigentlichen Druckwelle parallel auftreten wird, aber in vielen Fällen gar nicht wesentlich ist.

Wenn das distale Applikatorelement 25 gewechselt werden soll, schiebt der Benutzer das verschiebbare Gehäuseteil 29 nach rechts und damit die Klemmhülse 24 ebenfalls, sodass die Kugeln 27 radial nach außen gelangen können. Dabei sind übrigens die Öffnungen in dem Aufnahmestück 26 für die Kugeln 27 radial innen zu eng, sodass die Kugeln nicht herausfallen können.

Die Figuren 3 und 4 zeigen die Ausgestaltung zweier erfindungsgemäßer distaler Applikatorelemente für das Ausführungsbeispiel aus Figur 2. Figur 3 zeigt dabei eine Applikatorvariante 25', die abgesehen von der Anpassung an die Kupplung aus Figur 2 im Wesentlichen dem Applikator 9 aus Figur 1 entspricht. Sie ist allerdings etwas kürzer ausgeführt. Der Mehrkantstift 34 ist hierbei in ein Edelstahlteil 35 eingepresst, könnte aber vor allem bei Relevanz der eigentlichen hochfrequenten Schallwellen auch einstückig damit ausgeführt sein. Für den weichen Applikatorteil 36 gelten ansonsten die Ausführungen zu Figur 1.

Figur 4 zeigt eine Variante, bei der die konische Verjüngung des weichen Applikatorteils 36 weggelassen ist und der weiche Applikatorteil 37 stattdessen den Maximaldurchmesser bis zur Frontfläche beibehält. Die Frontfläche 38 hat einen flachen kreisringförmigen Außenbereich und eine sphärisch konkave Vertiefung darin. Letztere ist etwas größer, aber nicht unbedingt tiefer als die aus Figur 3; im gezeichneten Fall bleibt es aber bei dem Krümmungsradius von 8 mm.

Man kann sich bei beiden Ausführungsbeispielen leicht vorstellen, wie die Applikatorfrontfläche mit ihrer Vertiefung z. B. auf den Rücken eines Patienten im Wirbelbereich aufgesetzt und angedrückt werden kann und dabei infolge Formgebung und Materialwahl ergonomisch, verrutschsicher und mit gutem Flächenkontakt funktioniert. Dies bestätigen die Erprobungen der Erfinder.

## Patentansprüche

1. Gerät zur Behandlung des menschlichen oder tierischen Körpers mit
- einem Applikator (9, 11, 16, 22, 25, 25') zum Auflegen auf den Körper von außen,
- einem Gehäuse (1, 2, 3), in dem der Applikator (9, 11, 16, 22, 25, 25') gehalten ist, und
- einem Mechanismus (6, 13, 21) zum Erzeugen von Stößen des Applikators (9, 11, 16, 22, 25, 25') relativ zu dem Gehäuse (1, 2, 3) in einer Stoßrichtung, so dass die Stöße bei dem Auflegen in den Körper eingekoppelt werden können,
**dadurch gekennzeichnet, dass** der Applikator (9, 11, 16, 22, 25, 25') an einer in der Stoßrichtung nach vorn weisenden und zum Auflegen auf den Körper vorgesehenen Frontfläche (17, 38, 39) hohl und weich ausgestaltet ist und
die weiche Ausgestaltung sich daraus ergibt, dass der Applikator (9, 11, 16, 22, 25, 25') an der hohlen und weichen Frontfläche (17, 38, 39) aus einem Elastomermaterial mit einer Shore-Härte von höchstens 60 Sh besteht, wobei das Elastomermaterial an der Hohlform eine Stärke in der Stoßrichtung von mindestens 3 mm aufweist.

2. Gerät nach Anspruch 1, bei dem der Mechanismus (6, 13, 21) zum Erzeugen der Stöße ein Projektil (13) und eine Einrichtung (6, 21) zum Beschleunigen des Projektils (13) in solcher Weise aufweist, dass das Projektil (13) auf den Applikator (9, 11, 16, 22, 25, 25') schlägt und dadurch den Stoß erzeugt, vorzugsweise eine pneumatische Einrichtung zur Beschleunigung des Projektils (13).

3. Gerät nach Anspruch 2, bei dem der Applikator in dem Bereich (16, 22), den das Projektil (13) beim Aufschlagen trifft, aus einem nicht-elastomeren Material besteht und dieses Material in diesem Bereich eine Stärke in der Stoßrichtung von mindestens 2 mm hat.

4. Gerät nach Anspruch 1 oder 2, bei dem die Frontfläche (17, 38, 39) und vorzugsweise ein außerhalb des Gehäuses sichtbarer Teil des Applikators insgesamt rotationssymmetrisch um eine zur Stoßrichtung parallele Achse ist.

5. Gerät nach einem der vorstehenden Ansprüche, bei dem der Applikator (9, 11, 16, 22, 25) auf die Frontfläche (17, 38, 39) zu entlang der Stoßrichtung sich verjüngend geformt ist, vorzugsweise konisch.

6. Gerät nach einem der vorstehenden Ansprüche, bei dem das Material des Applikators (9, 11, 16, 22, 25, 25') mit der Shore-Härte von höchstens 60 Sh Silikonkautschuk ist.

7. Gerät nach Anspruch 3, optional in Verbindung mit Anspruch 4, 5 oder 6, bei dem der Applikator (9, 11, 16, 22, 25, 25') einen bezüglich der Stoßrichtung proximalen Teil (16, 35) und einen distalen Teil (11, 36, 37) aufweist, wobei der proximale Teil (16, 35) aus dem nicht-elastomeren Material besteht und der distale Teil (11, 36, 37) die Frontfläche (17, 38, 39) aufweist und der distale Teil (11, 36, 37) an oder in einer bezüglich der Stoßrichtung hinterschnittenen Form des proximalen Teils (16, 35) gehalten ist, vorzugsweise in einer hinterschnittenen Hohlform des proximalen Teils (16, 35) gehalten ist.

8. Gerät nach einem der vorstehenden Ansprüche, bei dem die Hohlform der Frontfläche (17, 38, 39) in der Stoßrichtung eine Tiefe von zwischen 10 % und 30 % des gemittelten Durchmessers der Hohlform senkrecht zur Stoßrichtung aufweist.

9. Gerät nach einem der vorstehenden Ansprüche, bei dem die Hohlform der Frontfläche (17, 38, 39) rundlich konkav ist, vorzugsweise sphärisch und vorzugsweise mit einem Krümmungsradius zwischen 5 mm und 20 mm.

10. Gerät nach einem der vorstehenden Ansprüche, bei dem die Hohlform der Frontfläche (17, 38, 39) von einer konvex, mit einem Radius von 0,5 mm bis 1,0 mm abgerundeten Kante umfasst ist.

11. Gerät nach einem der vorstehenden Ansprüche, bei dem das Material mit der Shore-Härte von höchstens 60 Sh andererseits eine Shore-Härte von mindestens 10 Sh aufweist.

12. Gerät nach einem der vorstehenden Ansprüche, bei dem der Applikator (22, 25, 25') in dem Gehäuse in einer Kupplung (27, 28, 29, 30) gehalten ist, wobei zumindest ein Applikatorelement (25, 25') nach Lösen der Kupplung (27, 28, 29, 30) in der Stoßrichtung aus dem Gehäuse herausziehbar ist.

13. Gerät nach Anspruch 12, bei dem der Applikator (22, 25, 25') zumindest zweiteilig ist, wobei ein distales Applikatorelement (25, 25') nach Lösen der Kupplung (27, 28, 29, 30) herausziehbar ist und ein proximales Applikatorelement (22) in dem Gehäuse verbleibt und wobei das proximale Applikatorelement (22) den Stoß auf das distale Applikatorelement (25, 25') überträgt.

14. Gerät nach einem der vorstehenden Ansprüche, das ausgelegt ist für einen Hub des Stoßes in der Stoßrichtung relativ zu dem Gehäuse (1, 2, 3) von mindestens 1 mm.

## Claims

1. An apparatus for treating the human or animal body, having
- an applicator (9, 11, 16, 22, 25, 25') for an extraneous application onto the body,
- a casing (1, 2, 3) in which the applicator (9, 11, 16, 22, 25, 25') is held, and
- a mechanism (6, 13, 21) for producing impacts of the applicator (9, 11, 16, 22, 25, 25') relative to the casing (1, 2, 3) in an impact direction so that the impacts can be coupled into the body in the application thereon,
**characterised in that** the applicator (9, 11, 16, 22, 25, 25') is made concave and soft at a front face (17, 38, 39) facing forwardly in the impact direction and adapted for being applied onto the body, and the softness results from that the applicator (9, 11, 16, 22, 25, 25') consists, at the concave and soft front face (17, 38, 39), of an elastomeric material having a Shore hardness of at most 60 Sh, wherein the elastomeric material has a thickness at the concave shape of at least 3 mm in the impact direction.

2. The apparatus of claim 1 wherein the mechanism (6, 13, 21) for producing the impacts comprises a projectile (13) and a device (6, 21) for accelerating the projectile (13) such that the projectile (13) impinges onto the applicator (9, 11, 16, 22, 25, 25') and thereby produces the impact, preferably a pneumatic device for accelerating the projectile (13).

3. The apparatus of claim 2 wherein the applicator consists, in the region (16, 22) being hit by the projectile (13) in the impinging, of a non-elastomeric material, and this material has a thickness in this region of at least 2 mm in the impact direction.

4. The apparatus of claim 1 or 2 wherein the front face (17, 38, 39) and preferrably a part of the applicator visible outside of the casing are rotationally symmetric, in total, with regard to an axis parallel to the impact direction.

5. The apparatus of one of the preceeding claims wherein the applicator (9, 11, 16, 22, 25) has a shape tapering towards the front face (17, 38, 39) along the impact direction, preferably a conical shape.

6. The apparatus of one of the preceeding claims wherein the material of the applicator (9, 11, 16, 22, 25, 25') having the Shore hardness of at most 60 Sh is silicone caoutchouc.

7. The apparatus of claim 3, optionally combined with claim 4, 5 or 6, wherein the applicator (9, 11, 16, 22, 25, 25') has, with regard to the impact direction, a proximal part (16, 35) and a distal part (11, 36, 37) wherein the proximal part (16, 35) consists of the non-elastomeric material and the distal part (11, 36, 37) comprises the front face (17, 38, 39), and the distal part (11, 36, 37) is held on or in a shape of the proximal part (16, 35) undercut with regard to the impact direction, preferably in an undercut concave shape of the proximal part (16,35).

8. The apparatus of one the preceeding claims wherein the concave shape of the front face (17, 38, 39) has a depth in the impact direction of between 10% and 30% of the average diameter of the concave shape perpendicular to the impact direction.

9. The apparatus of one of the preceeding claims wherein the concave shape of the front face (17, 38, 39) is concave in a rounded manner, preferably spherical and preferrably with a curvature radius between 5 mm and 20 mm.

10. The apparatus of one of the preceeding claims wherein the concave shape of the front face (17, 38, 39) is encompassed by a convex edge being rounded with a radius between 0.5 mm and 1.0 mm.

11. The apparatus of one of the preceeding claims wherein the material having the Shore hardness of at most 60 Sh on the other hand has a Shore hardness of at least 10 Sh.

12. The apparatus of one of the preceeding claims wherein the applicator (22, 25, 25') is held in the casing in a coupling device (27, 28, 29, 30), at least one applicator element (25, 25') being extractable from the casing in the impact direction after release of the coupling device (27, 28, 29, 30).

13. The apparatus of claim 12 wherein the applicator (22, 25, 25') has at least two elements wherein a distal applicator element (25, 25') is extractable after release of the coupling device (27, 28, 29, 30) and a proximal applicator element (22) remains in the casing, and wherein the proximal applicator element (22) transmits the impact onto the distal applicator element (25, 25').

14. The apparatus of one of the preceeding claims being adapted for a travel of the impact in the impact direction relative to the casing (1, 2, 3) of at least 1 mm.

## Revendications

1. Appareil de traitement du corps humain ou animal, comprenant:
- un applicateur (9, 11, 16, 22, 25, 25') destiné à une application externe sur ledit corps,
- un boîtier (1, 2, 3) dans lequel est retenu ledit applicateur (9, 11, 16, 22, 25, 25'), et
- un mécanisme (6, 13, 21) de production d'impacts de l'applicateur (9, 11, 16, 22, 25, 25') par rapport au boîtier (1, 2, 3) selon une direction d'impact, permettant de communiquer les impacts audit corps lors de l'application;
**caractérisé en ce que** l'applicateur (9, 11, 16, 22, 25, 25') présente une conception creuse et molle au niveau d'une face avant (17, 38, 39) dirigée vers l'avant, selon la direction d'impact, et destinée à être appliquée sur le corps, et
ladite conception molle provient du fait que l'applicateur (9, 11, 16, 22, 25, 25'), sur sa face avant (17, 38, 39) creuse et molle, se compose d'un matériau élastomère dont la dureté Shore ne dépasse pas 60 Sh;
ledit matériau élastomère présentant une épaisseur dans la direction d'impact, au niveau de la forme creuse, qui est d'au moins 3 mm.

2. Appareil selon la revendication 1, dans lequel le mécanisme (6, 13, 21) de production d'impacts présente un projectile (13) et un dispositif (6, 21) d'accélération du projectile (13) de sorte que le projectile (13) frappe sur l'applicateur (9, 11, 16, 22, 25, 25') pour ainsi produire l'impact, de préférence un dispositif pneumatique d'accélération du projectile (13).

3. Appareil selon la revendication 2, dans lequel l'applicateur se compose d'un matériau non élastomère dans la zone (16, 22) que vient frapper le projectile (13) lors de la percussion, et le matériau de cette zone présente une épaisseur d'au moins 2 mm dans la direction d'impact.

4. Appareil selon la revendication 1 ou 2, dans lequel la face avant (17, 38, 39) et, de préférence, une partie de l'applicateur visible en dehors du boîtier sont conjointement symétriques en rotation sur un axe parallèle à la direction d'impact.

5. Appareil selon l'une des revendications précédentes, dans lequel l'applicateur (9, 11, 16, 22, 25) va en s'effilant vers la face avant (17, 38, 39) le long de la direction d'impact, en étant de préférence conique.

6. Appareil selon l'une des revendications précédentes, dans lequel le matériau de l'applicateur (9, 11, 16, 22, 25, 25') de dureté Shore ne dépassant pas 60 Sh est du caoutchouc à base de silicone.

7. Appareil selon la revendication 3, facultativement en conjonction avec la revendication 4, 5 ou 6, dans lequel l'applicateur (9, 11, 16, 22, 25, 25') présente une partie proximale (16, 35) et une partie distale (11, 36, 37), eu égard à la direction d'impact ; ladite partie proximale (16, 35) se composant dudit matériau non élastomère et ladite partie distale (11, 36, 37) présentant ladite face avant (17, 38, 39), et ladite partie distale (11, 36, 37) étant retenue sur ou dans une forme de la partie proximale (16, 35) qui est contre-dépouillée eu égard à la direction d'impact, de préférence dans une forme creuse contre-dépouillée de la partie proximale (16, 35).

8. Appareil selon l'une des revendications précédentes, dans lequel la forme creuse de la face avant (17, 38, 39) présente, dans la direction d'impact, une profondeur comprise entre 10 % et 30 % du diamètre moyen de la forme creuse perpendiculairement à ladite direction d'impact.

9. Appareil selon l'une des revendications précédentes, dans lequel la forme creuse de la face avant (17, 38, 39) présente un arrondi concave, de préférence sphérique et de préférence avec un rayon de courbure compris entre 5 mm et 20 mm.

10. Appareil selon l'une des revendications précédentes, dans lequel la forme creuse de la face avant (17, 38, 39) est entourée d'un bord arrondi convexe d'un rayon allant de 0,5 mm à 1,0 mm.

11. Appareil selon l'une des revendications précédentes, dans lequel le matériau de dureté Shore ne dépassant pas 60 Sh présente par ailleurs une dureté Shore d'au moins 10 Sh.

12. Appareil selon l'une des revendications précédentes, dans lequel l'applicateur (22, 25, 25') est retenu dans le boîtier dans un dispositif de couplage (27, 28, 29, 30), au moins un élément d'applicateur (25, 25') pouvant être dégagé du boîtier après le désaccouplement du dispositif de couplage (27, 28, 29, 30) dans la direction d'impact.

13. Appareil selon la revendication 12, dans lequel l'applicateur (22, 25, 25') est constitué d'au moins deux pièces, moyennant quoi un élément d'applicateur distal (25, 25') peut être dégagé après le désaccouplement du dispositif de couplage (27, 28, 29, 30) et un élément d'applicateur proximal (22) demeure dans le boîtier, ledit élément d'applicateur proximal (22) transmettant l'impact sur l'élément d'applicateur distal (25, 25').

14. Appareil selon l'une des revendications précédentes, conçu pour permettre une course d'impact d'au moins 1 mm dans la direction d'impact par rapport au boîtier (1, 2, 3).
